# EUROPEAN PATENT APPLICATION

(11) **EP 3 384 948 A1**
(43) Date of publication of application: **10.10.2018**
(21) Application number: 17164862.9
(22) Date of filing: 04.04.2017
(51) Int. Cl.: A61M 16/00, A61M 16/20, G01F 25/00

(54) **AN AUTOMATED FLOW SENSOR CALIBRATION SYSTEM AND METHOD**

(71) Applicant: Medec Benelux NV, 9300 Aalst (BE)
(72) Inventor: BRAEM, Kristof, 9300 Aalst (BE)
(74) Representative: LC Patents

(57) **Abstract**

The invention relates to respiration systems (10) comprising a flow sensor (20) and a ventilator (30) for delivering gas to the patient (5) via the flow sensor (20). More particularly, the invention relates to calibrating the flow sensor (20) of such respiration system (10) in an automated manner, substantially improving the workflow, decreasing risk of human error and enhancing the quality of the obtained calibration data, including subsequent control and information capturing. A fully automated manner is further provided by not disconnecting the patient (5) while continuing ventilation.

## Description

### FIELD OF THE INVENTION

The invention relates to respiration systems comprising a flow sensor and a ventilator for delivering gas to the patient via the flow sensor. More particularly, the invention relates to calibrating the flow sensor of such respiration system, measuring gas flow and generating data to the respiration system. The gas flow measured and/or generated data may be used for determining specific information about the respiration, and/or for controlling the respiration.

### BACKGROUND TO THE INVENTION

Respiration systems with a flow sensor are known in the art. The calibration of such flow sensor is evenly described. However, the flow sensor itself has developed over the years, and is more recently and preferably designed for being placed in the vicinity of a human's mouth, further referred to as proximal flow sensor, wherein measurements can take place the closest possible to the patient's breathing activity, avoiding noise and/or errors. As an example of such proximal flow sensor, a differential pressure sensor can be used, wherein a difference in pressure is measured because of a physical restriction within the sensor system. When calibrating a proximal flow sensor, gas has to be led or flown through the sensor in both directions, meaning in the direction from patient to ventilator - further referred to as expiratory flow, but also in the opposite direction of gas delivered from the ventilator towards the patient i.e. from ventilator to patient - further referred to as inspiratory flow. During calibration, the flow sensor has to be dismounted - taken off the patient - and the breathing system partially disassembled, including reassembled in another configuration, within the respiration system, in order to perform the calibration by flowing gas in one direction and the other. Not only is such calibration process time consuming and cumbersome, it is moreover very sensitive to human errors as well as occurring noise and system errors due to the dismantling and reconnection of different parts. It would be an improvement, if the calibration could take place in a more automated manner, such that less time and occupation of medical staff is needed, and errors can be avoided. In addition, if a calibration procedure needs to be performed during ventilation, the patient needs to be disconnected and ventilated by other means, such as for instance a manual resuscitator or self-inflating bag. It would even be a further improvement if the calibration could occur without interrupting the ventilation of the patient.

### SUMMARY OF THE INVENTION

The invention provides for a respiration system performing the entire calibration without physically changing gas connections, and hence substantially improving the workflow, while decreasing risk of human error and enhancing the quality of the obtained calibration data, including subsequent control and information capturing.

A first aspect of the invention relates to a respiration system comprising a flow sensor, a ventilator for delivering a gas flow to the patient via said flow sensor and a 3-sided (Y-)piece, (indirectly) connecting (e.g. with tubes) said flow sensor (at least one side thereof) to said ventilator. Said respiration system is adapted for calibrating said flow sensor, characterized in that said respiration system further comprises a (selectable gas flow guiding) subsystem for (performing) an automated calibration of said flow sensor, more in particular said subsystem being further (indirectly) connectable or connected (e.g. with tubes) to said flow sensor (to the other side thereof). It is noted that said subsystem is connected to said flow sensor during operation of said automated calibration. According to an embodiment, said subsystem comprises a switching mechanism, i.e. comprising for instance (logic) ports and valves, for enabling the selective gas flow guidance for performing an automated calibration.

In a particular embodiment, said respiration system further comprises storage means like memory, to store (i) a first plurality of data values relating a gas flow with sensor data while said gas flow is delivered in a first direction, as used during a first part of the calibration, a second plurality of data values relating a gas flow with the sensor data while said gas flow is delivered in a second (reverse) direction, as used during a second part of the calibration.

In a particular embodiment, said respiration system further comprises computation means to determine control signals for said ventilator and/or for said (selectable gas flow guiding) subsystem, and/or information to be displayed or recorded based on said gas flow measured from said flow sensor.

A second aspect of the invention relates to a (selectable gas flow guiding) subsystem, being removably connectable to the respiration system of the first aspect of the invention; for (performing) an automated calibration of said flow sensor therein, more in particular said subsystem being further (indirectly) connectable or connected (e.g. with tubes) to the flow sensor therein.

A third aspect of the invention relates a method for calibrating a flow sensor of a respiration system as in the first aspect of the invention, comprising (i) the step of storing a first plurality of data values relating a gas flow with sensor data while said gas flow is delivered in a first direction, as used during a first part of the calibration, such as e.g. an inspiratory flow calibration while gas is flowing in the direction of the patient, (ii) the step of storing a second plurality of data values relating a gas flow with the sensor data while said gas flow is delivered in a second (reverse, e.g. opposite to the first) direction, as used during a second part of the calibration, such as e.g. an expiratory flow calibration while gas is flowing in the direction away from the patient.

In a particular embodiment of said third aspect, said method further comprises (iii) computing control signals for said ventilator and/or said (selectable gas flow guiding) subsystem to thereby provide and guide said gas flow in the required direction.

One or more of the above aspects can be combined.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.
**Fig. 1****: shows schematically the basic elements of a respiration system in accordance with the art.**
**Fig. 2****: illustrates the gas flow of the respiration system during inspiratory flow calibration part in accordance with the invention.**
**Fig. 3****: illustrates the gas flow of the respiration system during expiratory flow calibration part in accordance with the invention.**
**Fig. 4****: illustrates the gas flow connection during normal breathing operation.**
**Fig. 5****: illustrates an embodiment of the invention wherein the calibration is performed without disconnecting the respiration system from the patient.**
**Fig. 6****: illustrates the entire method of calibrating and normal operation of the respiration arrangement as supported with the various features of the invention.**
**Fig. 7****: illustrates the subsystem as separate device for retrofit solutions, more in particular illustrating the switching mechanism (a) for inspiratory flow calibration, and (b) for expiratory flow calibration.**
**Fig. 8****: illustrates an alternative embodiment of the invention wherein a switching mechanism for performing calibration in an automated manner is provided within the ventilator, more in particular (a) inspiratory flow calibration, and (b) expiratory flow calibration are shown. In (c) normal breathing operation of the system is depicted.**

### DETAILED DESCRIPTION OF THE INVENTION

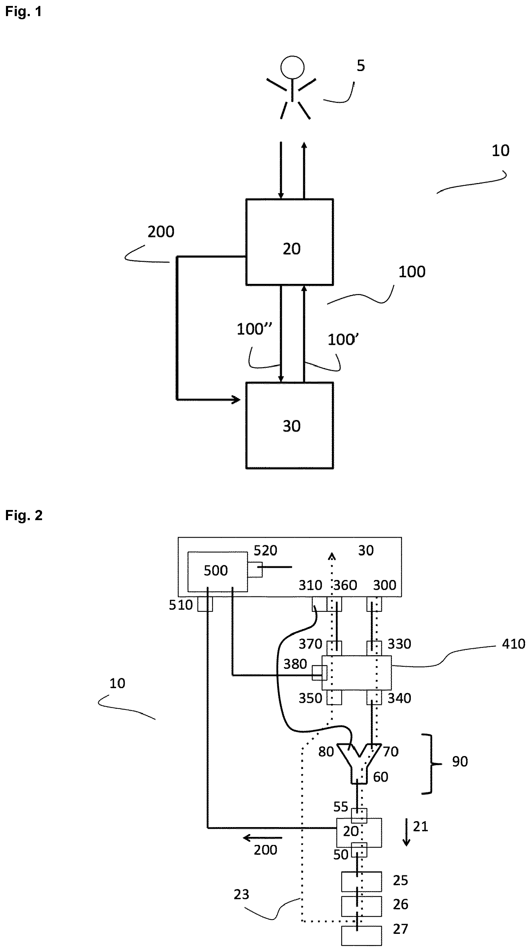

While referring to Figure 1, the invention relates to the field of respiration systems 10 known in the art, comprising a flow sensor 20, for example a proximal flow sensor, i.e. designed for being placed in the vicinity of a human's mouth, and a ventilator 30, for delivering a gas flow 100 towards a patient 5 via the flow sensor 20; wherein the flow sensor 20 measuring the gas flow 100, is generating data 200 to the ventilator 30 or the respiration system 10 in general. Hence, based on the gas flow measured by the flow sensor 20, the respiration system 10 may determine information about the respiration, or may control the respiration. The gas flow 100 can be in two directions: 100' for inspiratory and 100" for expiratory flow. A bidirectional gas flow is also illustrated between the patient 5 and the flow sensor 20. The gas mentioned usually comprises a mixture of oxygen and air, but may also include e.g. vapor and/or nitrous oxide. The data flow 200 may comprise a measurement signal, can be digital or analogue, and represents for example voltage or pressure values.

According to an embodiment of the invention, the gas flow sensor 20 as described above, comprises two ports as illustrated in Figure 2, wherein one port 50 is provided with a means designed for placement in the vicinity of a human's mouth - herewith referring again to a proximal flow sensor - and to thereby provide respiration to the human, and another port 55 is configured to be connected with a so-called Y-fitting or Y-piece 90. The port 50 may be fit for an adult person, or could be a neo-natal or pediatric port, or either is e.g. a hybrid port applicable for both adult and just born people. Such Y-piece 90, connecting the flow sensor 20 with the ventilator 30, for guiding inspiratory and expiratory flow, comprises a first channel 60 connected to the port 55 of the flow sensor 20, a second channel 70 the so-called inspiratory limb, designed for being connected to the ventilator 30 to thereby deliver gas to the patient, and a third channel 80 the so-called expiratory limb, to evacuate the gas breathed out by the patient back to the ventilator 30 via expiratory port 310. Although a subsystem 410 is provided between the Y-piece 90 and the ventilator 30, gas is indirectly (i.e. via the subsystem 410) delivered from inspiratory port 300 of the ventilator 30 to the Y-piece through the inspiratory limb 70, herewith passing inlet 330 and inspiratory port 340 of the subsystem 410.

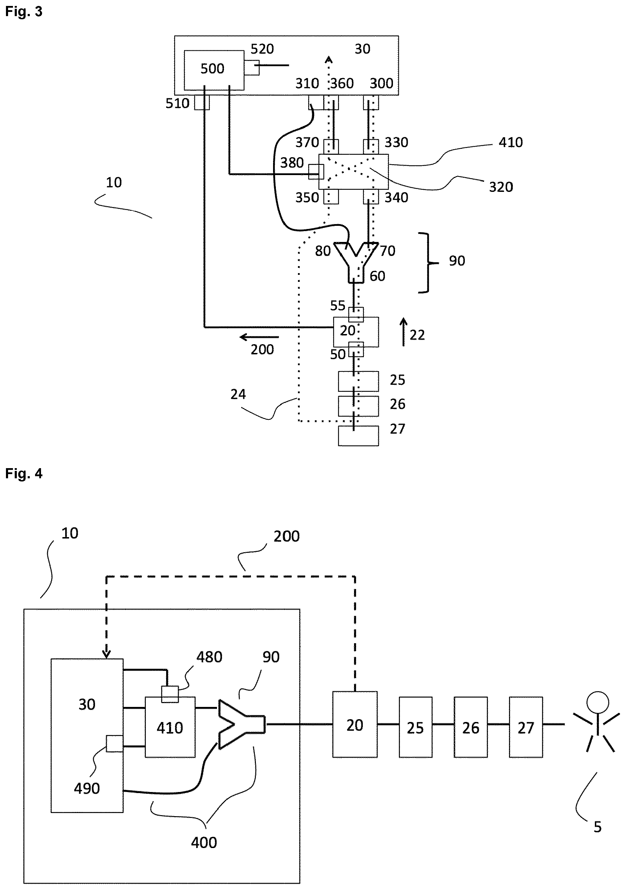

Further referring to Figure 2, the invention provides a respiration system 10 for delivering a gas flow towards a patient and passing a flow sensor 20, e.g. a proximal flow sensor, wherein the respiration system 10 is adapted for calibrating the flow sensor 20, and wherein the respiration system 10 comprises a Y-piece 90 and a ventilator 30. The ventilator 30 comprises three ports: a first ventilator port or inspiratory port 300 to indirectly (i.e. via the subsystem 410) connect to the second channel or inspiratory limb 70 of the Y-piece 90, to thereby deliver gas during normal operation and/or calibration, a second ventilator port or expiratory port 310 evacuating gas from the patient during normal operation - this gas being guided through the third channel or expiratory limb 80 of the Y-piece 90 back to the ventilator 30 - and a third port or test port 360 for evacuating gas that was guided through the flow sensor 20 during calibration. The subsystem 410 being connected to the Y-piece 90 on one hand and to the ventilator 30 on the other hand comprises of four ports: a first subsystem port or inlet 330 to connect to the inspiratory port 300 of the ventilator 30, guiding the delivered gas from the ventilator 30 during normal operation and/or calibration; a second subsystem port or inspiratory port 340 to connect to the second channel or inspiratory limb 70 of the Y-piece 90, further guiding the delivered gas from the ventilator 30 during normal operation and/or calibration or, as depicted in Figure 3, evacuating gas toward the ventilator 30 that was guided through the flow sensor 20 during calibration; a third subsystem port or test and calibration port 350 evacuating gas toward the ventilator 30 that was guided through the flow sensor 20 during calibration as illustrated in Figure 2 or, as depicted in Figure 3, further guiding the delivered gas from the ventilator 30 during calibration; a fourth subsystem port or outlet 370 further evacuating gas toward the ventilator 30 that was guided through the flow sensor 20 during calibration; and a fifth subsystem port or control port for controlling the switch operation.

During normal breathing operation as illustrated in Figure 4, gas flow delivered from the ventilator 30 passes the subsystem 410 and will subsequently enter the Y-piece 90 via its inspiratory limb 70, further guided through its first channel 60 followed by the flow sensor 20 and possibly other components 25 such as e.g. a capnostat (measuring CO₂ concentration), or an O₂ sensor, a filter 26 such as e.g. a HME (heat moisture exchanger) filter, or an anti-bacterial filter and/or a patient interface 27 such as for example a kind of mask, or an endotracheal tube, or a tracheostomy tube, towards the patient, whereas breathed out by the patient the gas is evacuated exiting the Y-piece 90 via its expiratory limb 80 towards the ventilator 30 and more particularly its expiratory port 310. Between the inspiratory port 340 and the inspiratory limb 70 a nebulizer and/or a humidifier (not shown) may also be provided. The data flow 200 comprising for instance a measurement signal as generated by the flow sensor 20 is delivered to the ventilator 30.

During calibration, more particularly the inspiratory flow calibration as illustrated in Figure 2, gas flow delivered from the ventilator 30 passes the subsystem 410 and will subsequently enter the Y-piece 90 via its inspiratory limb 70, further guided through its first channel 60 followed by the flow sensor 20 in a first direction 21, also referred to as inspiratory direction, and possibly other components 25 and a filter 26 as determined above being connected with the subsystem 410. Hence, the gas is evacuated entering the subsystem 410 via its test and calibration port 350 and its outlet 370 towards the ventilator 30 and more particularly its test port 360. The dotted line 23 in Figure 2 represents the entire inspiratory flow.

During calibration, more particularly the expiratory flow calibration as illustrated in Figure 3, gas flow delivered from the ventilator 30 passes the subsystem 410 and will subsequently enter a filter 26 and possibly other components 25 as determined above followed by the flow sensor 20 in a second direction 22, also referred to as expiratory direction, opposite to the first direction 21, whereas the gas is further guided through the Y-piece's 90 first channel 60 and exiting its inspiratory limb 70, being further evacuated entering the subsystem 410 via its inspiratory port 340 and its outlet 370 towards the ventilator 30 and more particularly its test port 360. As visualized in Figure 3 the gas flow during the expiratory flow calibration is apparently crossing within the subsystem 410, enabled by a switch 320 provided within this subsystem 410. The switch 320 will selectively guide gas delivered by the ventilator 30 either to the Y-piece's inspiratory limb 70, during normal breathing operation and/or an inspiratory flow calibration, or else to a filter 26 and possibly other components 25 as determined above followed by the flow sensor 20, during an expiratory flow calibration. The dotted line 24 in Figure 3 represents the entire expiratory flow.

During startup of the respiration system 10, the subsystem 410 can be tested by (automatically) checking whether the flow can take place in both inspiratory and expiratory direction.

In an embodiment, the invention provides a respiration system as described above, comprising a ventilator 30 and a breathing system 400, both capable of providing ordinary respiration capabilities, and further comprising a subsystem 410, removably connectable to the ventilator 30 and the breathing system 400, in case of service or maintenance activities. As shown in Figure 4, the breathing system 400 comprises a Y-piece 90 and connecting tubes.

According to an embodiment, the subsystem 410 of Figure 4 further comprises a control port 480 for controlling the switch operation by means of pneumatic and/or electric valve control, wherein the control port 480 is generally connected with the ventilator 30 for electrically or pneumatically driving the switch although another subsystem (not further specified here) within the respiration system 10 may also be configured for this functionality. The subsystem 410 hence comprises one or more valves for controlling the switching operation, such that automated calibration, defined by an inspiratory flow calibration part and an expiratory flow calibration part, can take place.

According to a particular embodiment, a cork valve 490 is integrated within the ventilator 30 either providing an occlusion for performing a leak test or to provide an exhaust path for ventilator tests.

Again referring to Figure 2, according to an embodiment of the invention the respiration system 10 and/or the ventilator 30 in particular, comprise electronic means 500, such as for instance storage means like memory and/or computation means, for storing and interpreting data 200, e.g. based on the measured gas flow, and/or determining the required gas flow (as input for the ventilator 30) and/or determining information about the respiration (to be provided to a display - not shown). In an embodiment, electronic input ports 510 (e.g. suitable for acquiring measurement signals coming from the flow sensor 20) and electronic output ports 520 (e.g. suitable for providing control signals to either the ventilator 30 and/or information to a display) are foreseen.

As mentioned, Figure 4 illustrates the gas flow connection during normal breathing operation.

During this normal breathing operation, the measurement signal (or data in case of a digital sensor) 200 is sensor data to be used to control the ventilator 30 and/or to generate to be displayed information to medical staff. During the inspiratory flow part of the calibration as depicted in Figure 2, the plurality of data values, i.e. the calibration data, is collected or captured and comprises data concerning the flow delivered by the ventilator 30 on one hand, as well as the measurement signal 200 generated by the flow sensor 20 on the other hand, possibly representing one or more different values. The plurality of data values is to be used for interpreting the measurement signal 200 and subsequently in the generating of suitable control signals or relevant to be displayed information. In Figure 3, the reversed gas flow direction and herewith provided switch connection characterizing the expiratory flow part of the calibration is shown. Especially note the entry of gas via the Y-piece first channel 60 in the direction of the gas flow directed from the flow sensor 20, i.e. referring to a flow direction of a patient breathing out during normal operation.

In a further embodiment the respiration system 10 is adapted i.e. having storage means like memory, to store (i) a first plurality of data values relating a flow with sensor data while the ventilator 30 delivered gas flows via the Y-piece 90 second channel or inspiratory limb 70 to the flow sensor 20 in a first direction 21, as determined during a first part of the calibration, (ii) a second plurality of data values relating a flow with the sensor data while the ventilator 30 delivered gas flows via the Y-piece 90 first channel 60 from the flow sensor 20 in a second direction 22, as determined during a second part of the calibration. Moreover, the respiration system 10 is adapted, i.e. having computation means e.g. to interpolate and/or integrate, to determine control signals for the ventilator 30 and/or information to be displayed or recorded (during normal breathing operation) based on measured data from the flow sensor 20.

In a further specific embodiment the flow sensor 20 and/or respiration system 10 is disclosed, wherein the flow sensor 20 is for instance a differential pressure sensor, a hot wire sensor, an ultrasound sensor or a CMOS heat sensor, generating each a particular type of data values to be mapped on gas flows. According to another embodiment, the flow sensor 20 is moreover a proximal flow sensor.

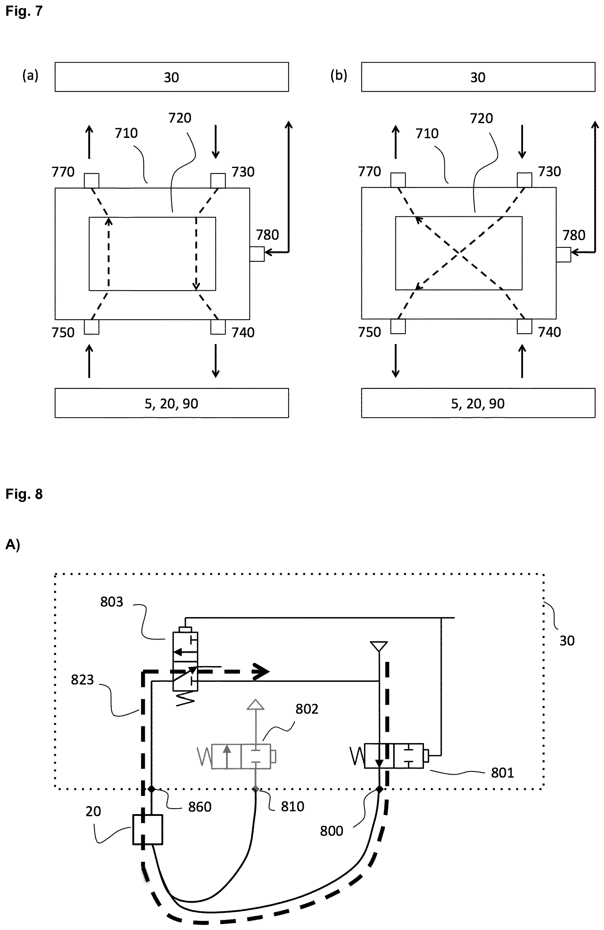

It is an aspect of the invention to provide a subsystem 710 as illustrated in Figure 7, removably connectable to a ventilator 30 and a breathing system 400, both capable of providing ordinary respiration capabilities and shown in Figure 4, wherein the ventilator 30 and the breathing system 400 together with the subsystem 710 jointly form a respiration system 10 suitable for a flow sensor 20 as discussed above. A switching mechanism or switch 720 is provided within the subsystem 710, selectively guiding gas delivered by the ventilator 30 either to the Y-piece's inspiratory limb 70, during normal breathing operation and/or an inspiratory flow of the calibration, or else to possibly other components 25 as previously specified followed by the flow sensor 20, during an expiratory flow of the calibration.

The subsystem 710 comprises five ports: a first subsystem port or inlet 730 to connect to the inspiratory port 300 of the ventilator 30, guiding the delivered gas from the ventilator 30 during normal operation and/or calibration; a second subsystem port or inspiratory port 740 to connect to the second channel or inspiratory limb 70 of the Y-piece 90, further guiding the delivered gas from the ventilator 30 during normal operation and/or calibration, or evacuating gas toward the ventilator 30 that was guided through the flow sensor 20 during calibration; a third subsystem port or test and calibration port 750 evacuating gas toward the ventilator 30 that was guided through the flow sensor 20 during calibration, or further guiding the delivered gas from the ventilator 30 during calibration; a fourth subsystem port or outlet 770 further evacuating gas toward the ventilator 30 that was guided through the flow sensor 20 during calibration; and a fifth subsystem port or control port 780, for controlling the switch operation by means of pneumatic and/or electric valve control, wherein the control port 780 is generally connected with the ventilator 30 for electrically or pneumatically driving the switch although another subsystem (not further specified here) within the respiration system 10 may also be configured for this functionality.

While referring to the switch 720 in particular, the above embodiment has the further advantage that hardware re-use in terms of the ventilator 30 is realized, while again an improvement of the quality of the obtained calibration data - and subsequent control and information capturing - is realized as the same ventilator 30 is used throughout the whole process.

Moreover, the above embodiment provides the additional advantage to re-use the entire subsystem 710 with its switch 720, with other ventilators and/or breathing systems.

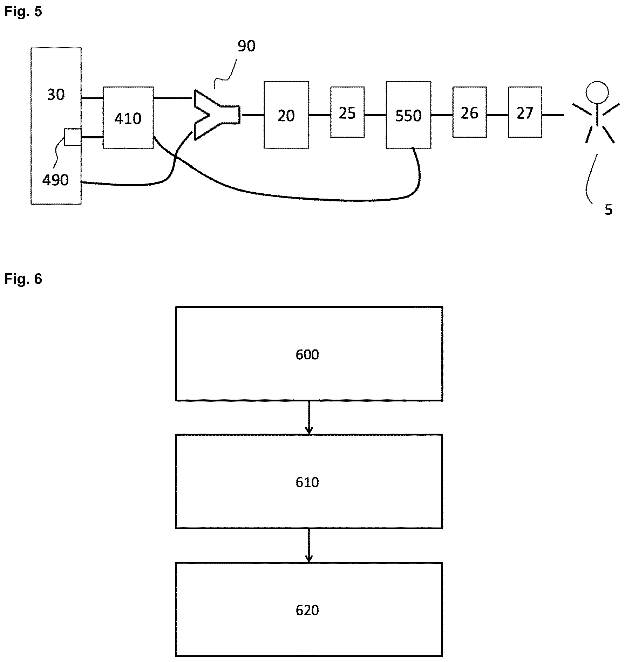

Figure 5 illustrates an embodiment of the invention, wherein the patient 5 remains connected to the respiration system 10 whenever calibration is performed. In other words, calibration and ventilation are occurring alternately. During normal ventilation or breathing operation, more particularly the inspiration, gas flow delivered from the ventilator 30 passes the subsystem 410 and will subsequently enter the Y-piece 90, followed by the flow sensor 20 in a first direction 21, also referred to as inspiratory direction, and possibly other components 25, filter 26 and patient interface 27 as determined earlier before finally reaching the patient 5. Moreover, during normal ventilation the cork valve 490 is closed, to avoid the evacuation of gas and close the exhaust path. During the expiration, gas flow exhaled by the patient 5 passes a patient interface 27, a filter 26 and other components 25 as determined earlier, followed by the flow sensor 20 in a second direction 22, also referred to as expiratory direction, and will subsequently enter the Y-piece 90 before arriving at the ventilator 30. A free breathing system 550 including one or more valves is provided between the components 25 and the patient 5 for controlling ventilation and calibration. Calibration can intermittently take place during expiration, meaning when gas is led back from the patient 5 to the ventilator 30. In case of inspiratory flow calibration, gas flow will be delivered from the ventilator 30 towards the subsystem 410, followed by the Y-piece 90, the flow sensor 20, other components 25 and free breathing system 550, and then returning back to the subsystem 410 and finally the ventilator 30. In case of expiratory flow calibration, gas flow will be delivered from the ventilator 30 towards the subsystem 410, followed by the free breathing system 550, other components 25, the flow sensor 20, the Y-piece 90, and again passing the subsystem 410 before finally arriving back at the ventilator 30. During calibration the patient 5 can freely inhale or exhale because of the free breathing system 550 being in the vicinity of the patient 5 while being connected with the subsystem 410 and thereby continuously provided with gas from the ventilator 30. Moreover, during calibration the cork valve 490 is open, in order to provide an exhaust path for the gas used during calibration.

According to a particular embodiment of the above, the free breathing system 500 comprises two three-way directional spool valves and a pressure relief valve.

Hence, in an embodiment, a closed system is provided wherein the gas flow is initially guided in a first direction, followed by a second direction within the same closed system.

Figure 6 illustrates the entire method of calibrating and normal operation of the respiration system 10 as supported with the various features of the invention, in particular in step 600 an inspiratory flow part of the calibration is performed while using the configuration as shown in Figure 2, whereas in step 610 an expiratory flow part of the calibration is performed while using the configuration as shown in Figure 3, and finally in step 620 the arrangement is used in its normal breathing operation using the configuration of Figure 4. One may iterate over these steps from time to time.

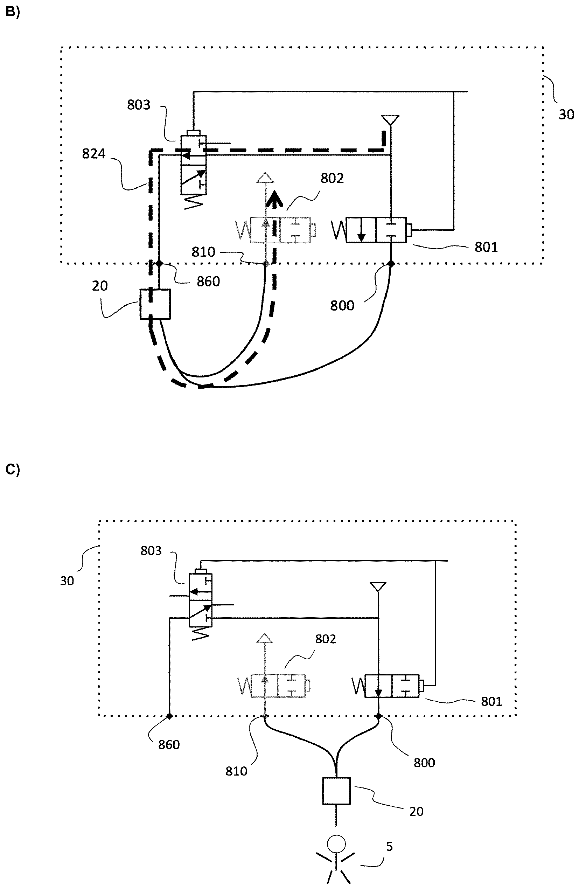

With Figure 8 a further embodiment is described, wherein instead of having a subsystem 410 wherein a switch 320 is provided, a switching mechanism is integrated within the ventilator 30 by means of commonly available valves on one hand and a less common (rather complex) switch valve on the other hand. One of the commonly available valves is for example a PEEP valve or expiration valve located at the expiratory port, while the other commonly available valve may be of the same type, hence may be a copy of the PEEP valve, and is provided in the neighborhood of the inspiratory port. The less common switch valve is positioned close to the test port. In Figure 8 (a) an example of a pneumatic circuit for the inspiratory flow calibration is shown, whereas Figure 8 (b) illustrates a corresponding possible circuit for the expiratory flow calibration. For completion, Figure 8 (c) represents the circuit for normal breathing operation of the system.

During inspiratory flow calibration of Figure 8 (a) gas is delivered via a first commonly available valve 801 within the ventilator 30 towards the inspiratory port 800 of the ventilator 30, and guided further outside the ventilator 30 through the flow sensor 20 towards the test port 860 of the ventilator 30 in order to reach the switch valve 803 within the ventilator 30. The dashed line 823 in Figure 8 (a) represents the entire gas flow during inspiratory flow calibration, while the arrow of this dashed line 823 indicates the gas flow direction.

During expiratory flow calibration of Figure 8 (b) gas is delivered via switch valve 803 within the ventilator 30 towards test port 860 of the ventilator 30, and guided further outside the ventilator 30 through the flow sensor 20 towards the expiratory port 810 of the ventilator 30 in order to reach a second commonly available valve 802 within the ventilator 30. The second commonly available valve 802 is usually known as a PEEP valve, wherein PEEP stands for 'positive end expiratory pressure'. The first commonly available valve 801 used during inspiratory flow calibration can be similar in type or even be a copy of the second commonly available valve 802. Both are standard, low in price and easily achievable, whereas the switch valve 803 is rather complex, and is therefore less common expensive. The dashed line 824 in Figure 8 (b) represents the entire gas flow during expiratory flow calibration, while the arrow of this dashed line 824 indicates the gas flow direction.

During normal breathing operation as illustrated in Figure 8 (c) gas is on one hand during inspiration delivered from the ventilator 30 to the patient 5 via the inspiratory port 800 while subsequently passing the flow sensor 20, whereas during expiration the flow is led from patient 5 to the ventilator 30 via the expiratory port 810.

## Claims

1. A respiration system (10) comprising a flow sensor (20), a ventilator (30) for delivering a gas flow (100) to a patient (5) via said flow sensor (20) and a Y-piece (90), connecting said flow sensor (20) to said ventilator (30), said respiration system (10) being adapted for calibrating said flow sensor (20), **characterized in that** said respiration system (10) further comprises a subsystem (410) for automated calibration of said flow sensor (20).

2. The respiration system (10) of claim 1, wherein said subsystem (410) further comprises a switch (320) for selectively guiding said gas flow (100) delivered by said ventilator (30).

3. The respiration system of claim 2, wherein said switch (320) of said subsystem (410) is selectively guiding said gas flow (100) delivered by said ventilator (30) either in a first direction (21) towards said Y-piece (90) followed by said flow sensor (20), or else in a second direction (22) towards said flow sensor (20) followed by said Y-piece (90).

4. The respiration system (10) of claim 3, wherein said first direction (21) is used during normal breathing operation and/or a first part of said automated calibration, and wherein said second direction (22) is used during a second part of said automated calibration.

5. The respiration system (10) of any of the preceding claims, wherein said flow sensor (20) measuring said gas flow (100) is generating data for said respiration system (10).

6. The respiration system (10) of any of the preceding claims, wherein said flow sensor (20) is a proximal flow sensor, more in particular a differential pressure sensor.

7. The respiration system (10) of any of the preceding claims, wherein said Y-piece (90) comprises a first channel (60) connected to said flow sensor (20), an inspiratory limb (70) connected to said subsystem (410), and an expiratory limb (80) connected to said ventilator (30).

8. The respiration system (10) of any of the preceding claims, wherein said ventilator (30) comprises a first ventilator port or inspiratory port (300), a second ventilator port or expiratory port (310), and a third ventilator port or test port (360).

9. The respiration system (10) of any of the preceding claims, wherein said subsystem (410) comprises a first subsystem port or inlet (330), a second subsystem port or inspiratory port (340), a third subsystem port or test and calibration port (350) and a fourth subsystem port or outlet (370).

10. The respiration system (10) of claim 9 wherein said subsystem (410) further comprises a control port (380) for controlling said subsystem (410) and/or further comprises electric means (500) for transferring data (200) to said respiration system (10) and/or determining information about the respiration.

11. The respiration system (10) of any of the preceding claims, further comprising a free breathing system (550) for an automated calibration **characterized in that** a patient (5) is not disconnected, while ventilation towards said patient (5) is continued.

12. A subsystem (410) for automated calibration of a flow sensor (20), said subsystem (410) being removably connectable to a respiration system (10) comprising a ventilator (30) delivering a gas flow (100) and a breathing system (400) comprising a Y-piece (90) and tubes, **characterized in that** said subsystem (410) comprises a switch (320) for selectively guiding said gas flow (100) delivered by said ventilator (30).

13. The subsystem (410) of claim 12, wherein said switch (320) is selectively guiding said gas flow (100) delivered by said ventilator (30) either in a first direction (21) towards said Y-piece (90) followed by said flow sensor (20), or else in a second direction (22) towards said flow sensor (20) followed by said Y-piece (90).

14. The subsystem (410) of claim 13, wherein said first direction (21) is defined during normal breathing operation and/or a first part of said automated calibration, and wherein said second direction (22) is defined during a second part of said automated calibration.

15. A method for calibrating a flow sensor (20) of a respiration system (10) comprising storage means like memory, to store (i) a first plurality of data values relating a gas flow (100) with sensor data (200) while said gas flow (100) is delivered in a first direction (21), as used during a first part of the calibration, (ii) a second plurality of data values relating a gas flow (100) with the sensor data (200) while said gas flow (100) is delivered in a second direction (22), as used during a second part of the calibration.
